# EUROPÄISCHE PATENTSCHRIFT

(11) **EP 0 653 396 B1**
(45) Veröffentlichungstag und Bekanntmachung des Hinweises auf die Patenterteilung: **12.02.1997**
(21) Anmeldenummer: 94117326.2
(22) Anmeldetag: 03.11.1994
(51) Int. Cl.: C07C 1/32, C07C 209/68, C07C 41/30, C07B 49/00

(54) **Verfahren zur Herstellung von 1,1'-Binaphthylen**
Process for the preparation of 1,1'-binaphthylene
Procédé pour la préparation de 1,1'-Binaphthylène

(30) Priorität: 13.11.1993 DE 4338826
(43) Veröffentlichungstag der Anmeldung: 17.05.1995
(73) Patentinhaber: HOECHST AKTIENGESELLSCHAFT, 65929 Frankfurt am Main (DE)
(72) Erfinder: Regnat, Dieter, Dr., D-60529 Frankfurt am Main (DE); Kleiner, Hans-Jerg, Dr., D-61476 Kronberg (DE)

(56) Entgegenhaltungen:
- EP-A- 0 326 286
- DATABASE WPI Section Ch, Week 9004, Derwent Publications Ltd., London, GB; Class B05, AN 90-026705 & JP-A-1 305 039 (SUMITOMO CHEM IND KK) 8. Dezember 1989
- DATABASE WPI Section Ch, Week 8742, Derwent Publications Ltd., London, GB; Class E14, AN 87-296427 & JP-A-62 209 040 (TOYO SODA MFG KK) 14. September 1987

## Beschreibung

Die vorliegende Erfindung betrifft ein Verfahren zur Herstellung von in 2,2'-Stellung substituierten 1,1'-Binaphthylen, die gegebenenfalls in einer oder mehreren der Stellungen 3 bis 7 und/oder 3' bis 7' Substituenten tragen können. Derartige 1,1'-Binaphthyle stellen Bausteine zur Herstellung einer Reihe von wichtigen Verbindungen dar, die in unterschiedlicher Weise Anwendung finden. So ist 2,2'-Dimethyl-1,1'-binaphthyl (vgl. Formel A) ein wichtiges Zwischenprodukt bei der Synthese von 2-zähnigen Biaryl-diphosphinen. Als ein Vertreter eines solchen 2-zähnigen Biaryl-diphosphins sei das 2,2'-Bis(diphenylphosphinomethyl)-1,1'-binaphthyl (="NAPHOS", siehe Formel B) aufgeführt.

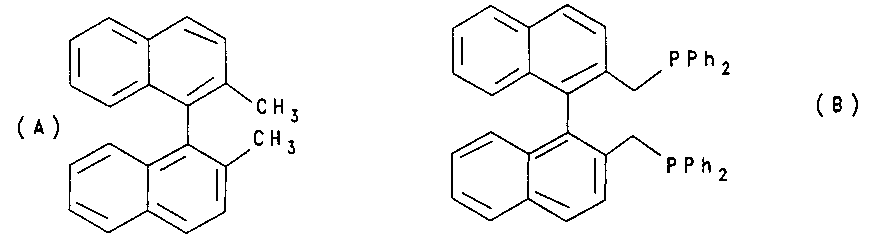

Verbindungen, die zwei Phosphinogruppen im Molekül enthalten (zweizähnige Phosphinliganden), spielen in einer Reihe von Verfahren, in denen ein Übergangsmetallkomplex als Katalysator Anwendung findet eine wichtige Rolle. Als Beispiele für derartige Verfahren seien Hydrierungs-, Hydroformylierungs-und Carbonylierungsreaktionen genannt.

Ein Weg zur Herstellung von in 2,2'-Stellung substituierten 1,1'-Binaphthylen, belegt am Beispiel von 2,2'-Dimethyl-1,1'-binaphthyl, besteht darin, 1-Brom-2-methyl-naphthalin und 2-Methyl-1-naphthylmagnesiumbromid in Gegenwart eines Nickel enthaltenden Katalysators (Nickel-katalysierte Aryl-kupplung) zu 2,2'-Dimethyl-1,1'-binaphthyl (siehe Formel A) umzusetzen. Da Nickel-Verbindungen aufgrund ihrer karzinogenen Eigenschaften ein gesundheitliches Risiko darstellen, kommen sie für einen technischen Einsatz nur mit starken Einschränkungen in Betracht. Ein weiterer, nicht unerheblicher Nachteil dieses Verfahrens (M. Kumada et al., Tetrahedron Lett. (1977), 1389) ist, daß diese Art der Synthese lediglich in Gemischen verschiedener Lösungsmittel wie Diethylether/Toluol oder Diethylether/Benzol zu Ausbeuten von bis zu 70 % zu dem gewünschten Wertprodukt führt. Die Handhabung derartiger Lösungsmittelgemische ist aufgrund der sehr hohen Flüchtigkeit und geringen Zündtemperatur von Diethylether problematisch. Ein zusätzliches Problem ergibt sich durch die Eigenschaft von Diethylether, unkontrolliert organische Peroxide zu bilden, die zu spontanen Explosionen führen können.
Es ist weiterhin bekannt, verschiedene in 2,2'-Stellung substituierte 1,1'-Binaphthyle durch Umsetzung entsprechend substituierter 1-Bromnaphthaline und 1-Naphthylmagnesiumbromide in Anwesenheit eines Palladium enthaltenden Katalysators (Palladium-katalysierte Arylkupplung) herzustellen. (T. Frejd und T. Klingstedt, Acta Chemica Scandinavica 43 (1989) 670). Bei der Herstellung von 2,2'-Dimethyl-1,1'-binaphthyl werden allerdings in Diethylether/Toluol nur mäßige Ausbeuten bis 50 % und in Toluol bei 80 °C nur sehr geringe Ausbeuten von 13 bis 30 % erzielt. Bei dieser Art der Synthese wird der Palladium-Katalysator jeweils in situ aus Palladium(II)-acetylacetonat und 2,2'-Dimethyl-6,6'-bis(diphenylphosphino)biphenyl als Diphosphin "BIPHEMP" (vergl. Formel C) oder 2,2'-Bis(diphenylphosphino)-1,1'-binaphthyl als Diphosphin "BINAP", (vgl. Formel D) erzeugt. Die eingesetzte Katalysatormenge beträgt jeweils 1 Mol-% bezogen auf 1-Brom-2-methylnaphthalin.

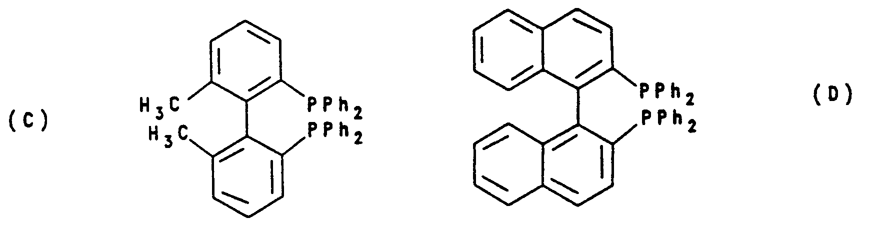

Im Hinblick auf die Verfahren des Standes der Technik stellt es eine sinnvolle Aufgabe dar, ein Verfahren zu entwickeln, das die vorstehend geschilderten Nachteile vermeidet und sich auf einfache Weise durchführen läßt.
Diese Aufgabe wird überraschenderweise gelöst durch ein Verfahren zur Herstellung von 1,1'-Binaphthylen. Es ist dadurch gekennzeichnet, daß man ein in 2-Stellung und gegebenenfalls in einer oder mehreren der Stellungen 3 bis 7 durch eine Alkyl-, Alkoxy- oder Dialkylaminogruppe mit 1 bis 6 Kohlenstoffatomen im Alkylrest substituiertes 1-Halogennaphthalin und ein in 2-Stellung und gegebenenfalls in einer oder mehreren der Stellungen 3 bis 7 durch eine Alkyl-, Alkoxy- oder Dialkylaminogruppe mit 1 bis 6 Kohlenstoffatomen im Alkylrest substituiertes 1-Naphthylmagnesiumhalogenid in einem unpolaren Lösungsmittel bei 30 bis 150 °C in Anwesenheit von Palladium und einem Phosphin der allgemeinen Formel 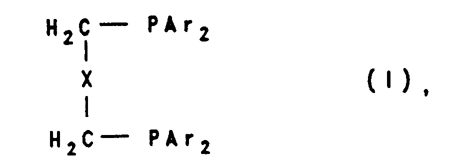 worin X für (CH₂)ₘ und m für eine ganze Zahl von 1 bis 4 oder X für einen Rest 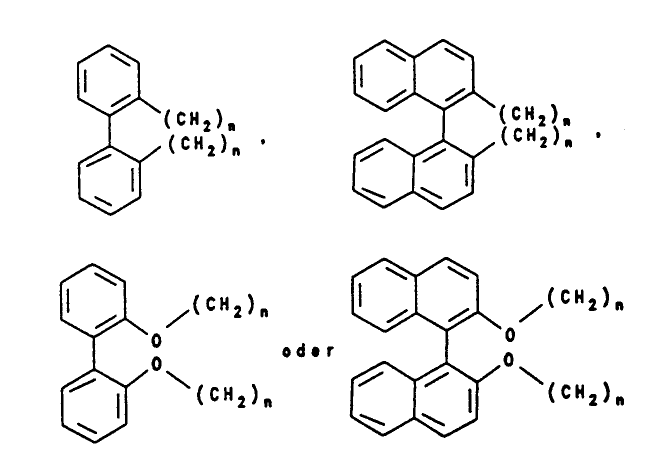 und n für eine ganze Zahl von 0 bis 2 steht, Ar unabhängig voneinander jeweils für einen Phenyl-, Tolyl-, Xylyl-, Fluorphenyl- oder Trifluormethylphenylrest steht, oder in Anwesenheit von Palladium und einem Phosphin der allgemeinen Formel 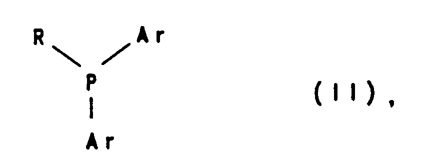 worin R für einen Alkylrest mit 1 bis 6 Kohlenstoffatomen, einen Cycloalkylrest mit 5 bis 6 Kohlenstoffatomen oder Ar steht und Ar jeweils die vorstehend genannte Bedeutung besitzt, umsetzt.

Bei Durchführung des erfindungsgemäßen Verfahrens ist darauf zu achten, daß - im Gegensatz zu T. Frejd, der die besten chemischen Ausbeuten erhält, wenn die Umsetzung in Diethylether gestartet wird - die Umsetzung von Anfang an in einem unpolaren Lösungsmittel, das auch von kleinen Mengen eines polaren Solvens oder eines Gemisches polarer Lösungsmittel frei ist, abläuft.

Ein Vorteil des erfindungsgemäßen Verfahrens besteht also darin, daß auf die Anwendung von Lösungsmittelgemischen verzichtet werden kann. Insbesondere ist es nicht erforderlich, in Anwesenheit von Diethylether zu arbeiten. Dadurch entfallen die im Umgang mit Diethylether auftretenden Risiken. Das erfindungsgemäße Verfahren läßt sich somit unter Verwendung üblicher Lösungsmittel auf einfache Weise und technisch sicher durchführen. Ein weiterer Vorteil des erfindungsgemäßen Verfahrens ist darin zu sehen, daß die gewünschten Wertprodukte in guten Ausbeuten zugänglich sind. Im Falle des 2,2'-Dimethyl-1,1'-binaphthyl lassen sich ohne weiteres Ausbeuten von 70 % und darüber erzielen.

Das erfindungsgemäße Verfahren läßt sich in einem vergleichweise breitem Umfange anwenden und führt zu in 2,2'-Stellung und gegebenenfalls in einer oder mehreren der Stellungen 3 bis 7 und/oder 3' bis 7' durch eine Alkyl-, Alkoxy- oder Dialkylaminogruppe mit jeweils 1 bis 6 Kohlenstoffatomen im Alkylrest substituierten 1,1'-Binaphthylen. Die Umsetzung verläuft nach dem folgenden, vereinfachten Reaktionschema. Der Einfachheit halber sind die in 3-bis 7 bzw. 3' bis 7' denkbaren Substituenten nicht mit aufgeführt.

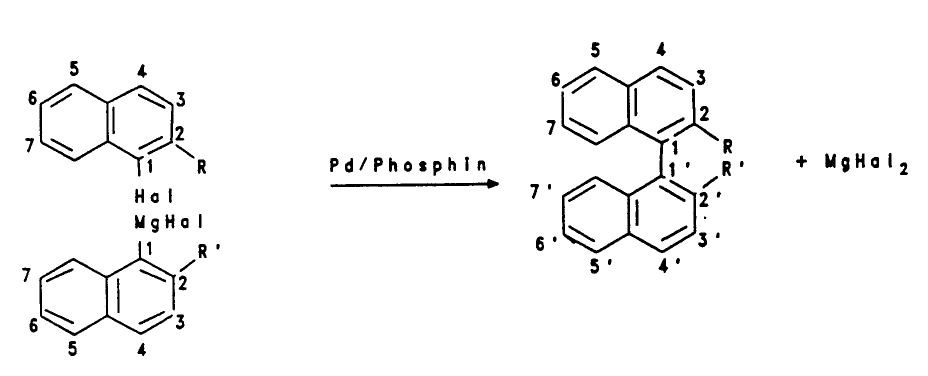

Als substituiertes 1-Halogennaphthalin setzt man ein in 2-Stellung und gegebenenfalls in einer oder mehreren der Stellungen 3 bis 7 durch eine Alkyl-Alkoxy oder Dialkylaminogruppe mit 1 bis 6 Kohlenstoffatomen im Alkylrest substituiertes 1-Halogennaphthalin ein. Als Beispiele hierfür seien 1-Brom-2-methylnaphthalin, 1-Brom-2-ethylnaphthalin, 1-Brom-4-dimethylamino-2-methylnaphthalin und 1-Brom-4-methoxi-2-methylnaphthalin genannt.

Gut geeignet als substituiertes 1-Halogennaphthalin sind 2-Alkyl-1-halogennaphthaline mit jeweils 1 bis 6 Kohlenstoffatomen im Alkylrest, insbesondere 2-Methyl-1-halogennaphthalin.

Üblicherweise setzt man als substituiertes 1-Halogennaphthalin ein substituiertes 1-Bromnaphthalin oder ein substituiertes 1-Jodnaphthalin, insbesondere ein substituiertes 1-Bromnaphthalin ein.

Als substituiertes Naphthylmagnesiumhalogenid setzt man ein in 2-Stellung und gegebenenfalls in einer oder mehreren der Stellungen 3 bis 7 durch eine Alkyl-, Alkoxy- oder Dialkylaminogruppe mit jeweils 1 bis 6 Kohlenstoffatomen im Alkylrest substituiertes 1-Naphthylmagnesiumhalogenid ein. Diese Verbindungen lassen sich durch Umsetzung eines entsprechend substituierten 1-Halogennaphthalins mit Magnesium (Grignard-Reaktion) herstellen, wobei in der Regel ein Ether, beispielsweise Diethylether, als Lösungsmittel Anwendung findet. Nach Abschluß der Grignard-Reaktion setzt man der Ether enthaltenden Lösung ein unpolares Lösungsmittel zu und destilliert anschließend den Ether ab. Man erhält auf diese Weise ein etherfreies Grignard-Reagenz. Ohne den Anspruch auf Vollständigkeit zu erheben seien als Beispiele für substituierte 1-Naphthylmagnesiumhalogenide 2-Methyl-1-naphthylmagnesiumbromid, 2-Ethyl-1-naphthylmagnesiumbromid, 4-Dimethylamino-2-methyl-1-naphthylmagnesiumbromid und 4-Methoxy-2-methyl-1-naphthylmagnesiumbromid genannt.

Gut geeignet als substituiertes 1-Naphthylmagnesiumhalogenid sind 2-Alkyl-1-naphthylmagnesiumhalogenide mit jeweils 1 bis 6 Kohlenstoffatomen im Alkylrest, insbesondere 2-Methyl-1-naphthylmagnesiumhalogenid.

Im allgemeinen verwendet man als substituiertes 1-Naphthylmagnesiumhalogenid ein substituiertes 1-Naphthylmagnesiumbromid oder ein substituiertes 1-Naphthylmagnesiumjodid, insbesondere ein substituiertes 1-Naphthylmagnesiumbromid.
Zur Durchführung der Reaktion setzt man das substituierte 1-Halogennaphthalin und das substituierte 1-Naphthylmagnesiumhalogenid im Mol-Verhältnis 1:(0,8 bis 1,2), insbesondere 1:(0,9 bis 1,1) um. In einer Reihe von Fällen hat es sich als ausreichend erwiesen, das substituierte 1-Halogennaphthalin und das substituierte 1-Naphthylmagnesiumhalogenid in äquimolaren Mengen reagieren zu lassen.

Man führt die Reaktion In Anwesenheit eines unpolaren Lösungsmittels durch. Geeignet als unpolares Lösungsmittel sind Toluol, Ethylbenzol, o-Xylol, m-Xylol, p-Xylol, technische Gemische isomerer Xylole oder auch Mischungen der vorstehend genannten Lösungsmittel.

Die Umsetzung des substituierten 1-Halogennaphthalins und des substituierten 1-Naphthylmagnesiumhalogenids läßt sich in einem relativ breiten Temperaturbereich von 30 bis 150 °C durchführen. Für ein Reihe von Fällen hat es sich als günstig erwiesen, die Umsetzung bei 50 bis 120, insbesondere 70 bis 100 °C durchzuführen.

Die Umsetzung erfolgt in Anwesenheit von Palladium. Üblicherweise setzt man das Palladium entsprechend einem Molverhältnis von substituiertem 1-Halogennaphthalin zu Palladium von (500 bis 20):1 insbesondere (200 bis 50):1 ein.
Geeignet als Palladium sind Palladium(II)-Verbindungen oder Palladium(0)-Komplexe.
Als Beispiele für Palladium(II)-Verbindungen seien Palladium(II)-acetat, Palladium(II)-sulfat, Palladium(II)-nitrat, Palladium(II)-chlorid, und/oder Palladium(II)-acetylacetonat genannt. Als Beispiel für einen Palladium(0)-Komplex steht Palladium-bis(dibenzylidenaceton).

Die Umsetzung wird in Anwesenheit eines Phosphins der allgemeinen Formel (I) durchgeführt. Gut geeignete Phosphine der allgemeinen Formel (I) sind solche, in denen m für eine ganze Zahl von 1 bis 4, insbesondere 1 bis 2, und n für eine ganze Zahl von 0 bis 2 und Ar unabhängig voneinander für einen Phenyl-, Tolyl-oder Fluorphenylrest steht.
Beispiele für derartige Phosphine der allgemeinen Formel (I) sind
1,4-Bis(diphenylphosphino)butan, 1,3-Bis(diphenylphosphino)propan,
2,2'-Bis(diphenylphosphinomethoxi)biphenyl oder
2,2'-Bis(diphenylphosphinomethoxi)-1,1'-binaphthyl, insbesondere
1,3-Bis(diphenylphosphino)propan, 1,4-Bis(diphenylphosphino)butan oder
2,2'-Bis(diphenylphosphinomethoxi)-1,1'-binaphthyl, bevorzugt
2,2'-Bis(diphenylphosphinomethoxi)-1,1'-binaphthyl.

Anstelle eines Phosphins der allgemeinen Formel (I) läßt sich auch ein Phosphin der allgemeinen Formel (II) mit gutem Erfolg einsetzen. Gut geeignete Phosphine der allgemeinen Formel (II) sind solche, in denen R für Ar und Ar für einen Phenyl-, Tolyl oder Fluorphenylrest steht. Besonders geeignet ist Triphenylphosphin oder Tri-p-tolylphosphin, insbesondere Triphenylphosphin.

Man setzt Palladium, beispielsweise eine Pd(II)-Verbindung, und das Phosphin der Formel (I) im Mol-Verhältnis 1:(0,8 bis 2,0), insbesondere 1:(0,9 bis 1,5) ein. In den meisten Fällen genügt es, Palladium und das Phosphin der Formel (I) im Mol-Verhältnis 1:1 zu verwenden.

Gebraucht man anstelle des Diphosphins der Formel (I) ein Monophosphin der Formel (II), so setzt man Palladium und das Phosphin der Formel (I) im MolVerhältnis 1:(1,6 bis 4,0), insbesondere 1:(1,8 bis 3,0) ein. In den meisten Fällen erweist sich ein Molverhältnis Palladium zu Phosphin der Formel (II) von 1:2 als ausreichend.

Das erfindungsgemäße Verfahren läßt sich besonders einfach durchführen, wenn man anstelle von Palladium und dem Phosphin einen Palladium-Komplex, der bereits ein Phosphin der allgemeinen Formel (I) oder der allgemeinen Formel (II) enthält, verwendet. Als derartige Palladium-Verbindungen kommen beispielsweise Palladium-tetrakis-triphenylphosphin, Palladium-bis-[2,2'-bis(diphenylphosphinomethoxi)-1,1'-binaphthyl] und Palladium-bis-[1,3-bis(diphenylphosphino)propan] in Betracht.

Die Umsetzung erfolgt im allgemeinen durch Zugabe des substituierten 1-Naphthylmagnesiumhalogenids (Grignard-Verbindung) zu einer in einem unpolaren Lösungsmittel hergestellten Suspension, respektive Lösung, von substituiertem 1-Halogennaphthalin, der Palladiumverbindung und dem Phosphin der allgemeinen Formel (I) oder (II), beziehungsweise dem das Phosphin der allgemeinen Formel (I) oder (II) enthaltenden Palladium(0)-Komplex. Die Aufarbeitung erfolgt anschließend durch Hydrolyse mit Wasser und verdünnter Säure, insbesondere verdünnter Mineralsäure. Das gewünschte Wertprodukt wird nach Abdestillieren des Lösungsmittel durch Destillation im Hochvakuum oder durch Umkristallisieren gewonnen.

Die nachstehenden Beispiele belegen die Erfindung, ohne sie darauf zu beschränken.

### Experimenteller Teil

### Beispiel 1

### Herstellung von 2-Methyl-1,-naphthylmagnesiumbromid

Unter Ausschluß von Luft und Feuchtigkeit wird zu einer Suspension von 3,7 g (0,15 Mol) Magnesiumspäne in 10 ml wasserfreiem Diethylether und 10 ml wasserfreiem Toluol eine Lösung von 31,0 g (0,14 Mol) 1-Brom-2-methylnaphthalin in 50 ml wasserfreiem Diethylether und 50 ml wasserfreiem Toluol zugetropft. Anschließend erhitzt man 6 Stunden zum Rückfluß und destilliert anschließend den Diethylether ab.

### Beispiel 2

### Herstellung von 2,2'-Dimethyl-1,1'-binaphthyl

Unter Ausschluß von Luft und Feuchtigkeit wird das entsprechend Beispiel 1 hergestellte 2-Methyl-1-naphthylmagnesiumbromid in Toluol einer auf 70 bis 80 °C erwärmten Suspension von 31,0 g (0,14 Mol) 1-Brom-2-methylnaphthalin, (0,0014 Mol) Phosphin der allgemeinen Formel (I) [Bis-1,4-(diphenylphosphino)butan; 2,2'-Bis-(diphenylphospinomethoxi)-1,1'-binaphthyl] oder 0,0028 Mol Phosphin der allgemeinen Formel (II) (Triphenylphosphin) und 0,31 g (0,0014 Mol) Palladium(II)-acetat in 50 ml Toluol zugesetzt. Man erwärmt anschließend noch eine Stunde auf 80 °C, kühlt auf 25 °C ab und gibt nacheinander 100 ml Wasser und 50 ml 2-n-Salzsäure zu. Man trennt die Phasen, extrahiert die wäßrige Phase mit 50 ml Toluol und engt die vereinigten organischen Phasen im Vakuum ein. Nach Destillation des öligen, braunen Rückstandes im Hochvakuum erhält man ein blaßgelbes Öl mit einem Siedepunkt von 175 bis 180 °C/0,1 bis 0,2 mbar. Die Ausbeuten sind der nachfolgenden Tabelle 1 zu entnehmen.

**Tabelle 1**

| Ausbeuten gemäß Beispiel 2 | |
|---|---|
| Phosphinligand der Formel (I) oder (II) | Ausbeute [%] |
| Triphenylphosphin | 77 |
| Bis-1,4-(diphenylphosphino)butan | 79 |
| 2,2'-Bis-(diphenylphosphinomethoxi)-1,1'-binaphthyl | 80 |

### Beispiel 3

### Herstellung von 2,2'-Dimethyl-1,1'-binaphthyl

Unter Ausschluß von Luft und Feuchtigkeit wird das entsprechend Beispiel 1 hergestellte 2-Methyl-1-naphthylmagnesiumbromid in Toluol einer auf 70 bis 80 °C erwärmten Lösung von 31,0 g (0,14 Mol) 1-Brom-2-methylnaphthalin und 1,6 g (0,0014 Mol) Palladium(0)-tetrakis-triphenylphosphin in 50 ml Toluol zugesetzt. Man erwärmt anschließend noch 1 Stunde auf 80 °C, kühlt auf 25 °C ab und gibt nacheinander 100 ml Wasser und 50 ml 2n-Salzsäure zu. Man trennt die Phasen, extrahiert die wässrige Phase mit 50 ml Xylol und engt die vereinigten organischen Phasen im Vakuum ein. Nach Destillation des öligen, braunen Rückstandes im Hochvakuum erhält man 30,8 g (78 %) blaßgelbes, zähes Öl mit einem Siedepunkt von 175 bis 180 °C/0,1 bis 0,2 mbar.

## Patentansprüche

1. Verfahren zur Herstellung von 1,1'-Binaphthylen, in dem man ein in 2-Stellung und gegebenenfalls in einer oder mehreren der Stellungen 3 bis 7 durch eine Alkyl-, Alkoxy- oder Dialkylaminogruppe mit 1 bis 6 Kohlenstoffatomen im Alkylrest substituiertes 1-Halogennaphthalin und ein in 2-Stellung und gegebenenfalls in einer oder mehreren der Stellungen 3 bis 7 durch eine Alkyl-, Alkoxy- oder Dialkylaminogruppe mit 1 bis 6 Kohlenstoffatomen im Alkylrest substituiertes 1-Naphthylmagnesiumhalogenid in einem unpolaren Lösungsmittel bei 30 bis 150 °C in Anwesenheit von Palladium und einem Phosphin der allgemeinen Formel 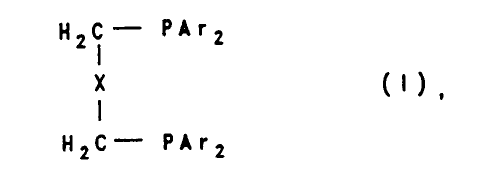 worin X für (CH₂)ₘ und m für eine ganze Zahl von 1 bis 4 oder X für einen Rest 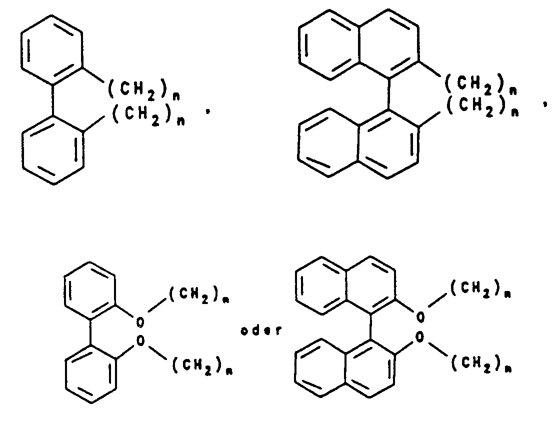 und n für eine ganze Zahl von 0 bis 2 steht, Ar unabhängig voneinander jeweils für einen Phenyl-, Tolyl-, Xylyl-, Fluorphenyl- oder Trifluormethylphenylrest steht, oder in Anwesenheit von Palladium und einem Phosphin der allgemeinen Formel 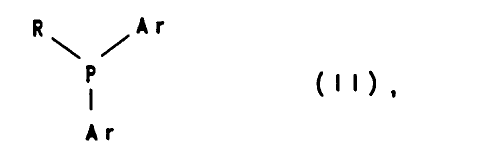 worin R für einen Alkylrest mit 1 bis 6 Kohlenstoffatomen, einen Cycloalkylrest mit 5 bis 6 Kohlenstoffatomen oder Ar steht und Ar jeweils die vorstehend genannte Bedeutung besitzt, umsetzt.

2. Verfahren nach Anspruch 1, dadurch gekennzeichnet, daß man als substituiertes 1-Halogennaphthalin ein 2-Alkyl-1-halogennaphthalin mit 1 bis 6 Kohlenstoffatomen im Alkylrest einsetzt.

3. Verfahren nach Anspruch 1 oder 2, dadurch gekennzeichnet, daß man als substituiertes 1-Halogennaphthalin ein 2-Methyl-1-halogennaphthalin einsetzt.

4. Verfahren nach einem oder mehreren der Ansprüche 1 bis 3, dadurch gekennzeichnet, daß man als substituiertes 1-Halogennaphthalin ein substituiertes 1-Bromnaphthalin oder 1-Jodnaphthalin einsetzt.

5. Verfahren nach einem oder mehreren der Ansprüche 1 bis 4, dadurch gekennzeichnet, daß man als substituiertes 1-Naphthylmagnesiumhalogenid ein 2-Alkyl-1-naphthylmagnesiumhalogenid mit 1 bis 6 Kohlenstoffatomen im Alkylrest einsetzt.

6. Verfahren nach einem oder mehreren der Ansprüche 1 bis 5, dadurch gekennzeichnet, daß man als substituiertes 1-Naphthylmagnesiumhalogenid ein 2-Methyl-1-naphthalinmagnesiumhalogenid einsetzt.

7. Verfahren nach einem oder mehreren der Ansprüche 1 bis 6, dadurch gekennzeichnet, daß man als substituiertes 1-Naphthylmagnesiumhalogenid ein substituiertes 1-Naphthylmagnesiumbromid oder 1-Naphthylmagnesiumjodid einsetzt.

8. Verfahren nach einem oder mehreren der Ansprüche 1 bis 7, dadurch gekennzeichnet, daß man das substituierte 1-Halogennaphthalin und das substituierte 1-Naphthylmagnesiumhalogenid im Mol-Verhältnis 1:(0,8 bis 1,2), insbesondere 1:(0,9 bis 1,1) umsetzt.

9. Verfahren nach einem oder mehreren der Ansprüche 1 bis 8, dadurch gekennzeichnet, daß man als unpolares Lösungsmittel Toluol, Ethylbenzol, o-Xylol, m-Xylol, p-Xylol, technische Gemische isomerer Xylole oder Mischungen derselben einsetzt.

10. Verfahren nach einem oder mehreren der Ansprüche 1 bis 9, dadurch gekennzeichnet, daß man die Umsetzung bei 50 bis 120, insbesondere 70 bis 100 °C durchführt.

11. Verfahren nach einem oder mehreren der Ansprüche 1 bis 10, dadurch gekennzeichnet, daß man das Palladium entsprechend einem Molverhältnis von substituierten 1-Halogennaphthalin : Palladium von (500 bis 20):1, insbesondere (200 bis 50):1 einsetzt.

12. Verfahren nach einem oder mehreren der Ansprüche 1 bis 11, dadurch gekennzeichnet, daß man als Palladium eine Pd(II)-Verbindung einsetzt.

13. Verfahren nach einem oder mehreren der Ansprüche 1 bis 12, dadurch gekennzeichnet, daß man Pd(II)-acetat, Pd(II)-sulfat, Pd(II)-nitrat, Pd(II)-chlorid, Pd(II)-acetylacetonat als Pd(II)-Verbindung einsetzt.

14. Verfahren nach einem oder mehreren der Ansprüche 1 bis 13, dadurch gekennzeichnet, daß man ein Phosphin der allgemeinen Formel (I), worin m für eine ganze Zahl von 1 bis 4, insbesondere 1 bis 2, und n für eine ganze Zahl von 0 bis 2 steht, einsetzt.

15. Verfahren nach einem oder mehreren der Ansprüche 1 bis 14, dadurch gekennzeichnet, daß man ein Phosphin der allgemeinen Formel (I), worin Ar unabhängig voneinander jeweils für einen Phenyl-, Tolyl- oder Fluorphenylrest steht, einsetzt.

16. Verfahren nach einen oder mehreren der Ansprüche 1 bis 15, dadurch gekennzeichnet, daß man als Phosphin der allgemeinen Formel (I) 1,4-Bis(diphenylphosphino)butan, 1,3-Bis(diphenylphosphino)propan, 2,2'-Bis(diphenylphosphinomethoxi)biphenyl oder 2,2'-Bis(diphenylphosphinomethoxi)-1,1'-binaphthyl, insbesondere 2,2'-Bis(diphenylphosphinomethoxi)-1,1'-binaphthyl einsetzt.

17. Verfahren nach einem oder mehreren der Ansprüche 1 bis 13, dadurch gekennzeichnet, daß man ein Phosphin der allgemeinen Formel (II), worin R für Ar und Ar für einen Phenyl-, Tolyl- oder Fluorphenylrest steht, einsetzt.

18. Verfahren nach einem oder mehreren der Ansprüche 1 bis 13, dadurch gekennzeichnet, daß man als Phosphin der allgemeinen Formel (II) Triphenylphosphin oder Tri-p-tolylphosphin einsetzt.

19. Verfahren nach einem oder mehreren der Ansprüche 1 bis 18, dadurch gekennzeichnet, daß man Palladium und Phosphin der Formel (I) im Molverhältnis 1:(0,8 bis 2,0), insbesondere 1:(0,9 bis 1,5), oder Palladium und Phosphin der Formel (II) im Molverhältnis 1:(1,6 bis 4,0), insbesondere 1:(1,8 bis 3,0) einsetzt.

20. Verfahren nach einem oder mehreren der Ansprüche 1 bis 19, dadurch gekennzeichnet, daß man Palladium und das Phosphin der Formel (I) oder (II) in Form eines das Phosphin der Formel (I) oder (II) enthaltenden Palladium-Komplexes einsetzt.

21. Verfahren nach einem oder mehreren der Ansprüche 1 bis 20, dadurch gekennzeichnet, daß man das Palladium und das Phosphin der Formel (I) oder (II) in Form von Palladium-bis-[1,3-bis(diphenylphosphino)propan], Palladium-bis-[2,2'-bis(diphenylphosphinomethoxi)-1,1'-binaphthyl] oder Palladium-tetrakis-triphenylphosphin einsetzt.

## Claims

1. A process for preparing 1,1'-binaphthyls, which comprises reacting a 1-halonaphthalene substituted in the 2 position and, if desired, in one or more of positions 3 to 7 by an alkyl, alkoxy or dialkylamino group having 1 to 6 carbon atoms in the alkyl radical and a 1-naphthylmagnesium halide substituted in the 2 position and, if desired, in one or more of positions 3 to 7 by an alkyl, alkoxy or dialkylamino group having 1 to 6 carbon atoms in the alkyl radical in a nonpolar solvent at 30 to 150°C in the presence of palladium and a phosphine of the formula 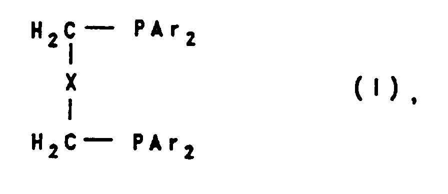 in which X is (CH₂)ₘ and m is an integer from 1 to 4 or X is a radical 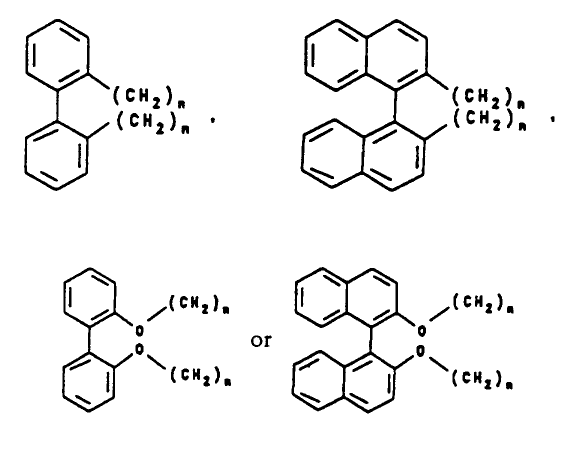 and n is an integer from 0 to 2, each Ar is, independently of the others, a phenyl, tolyl, xylyl, fluorophenyl or trifluoromethylphenyl radical, or in the presence of palladium and a phosphine of the formula 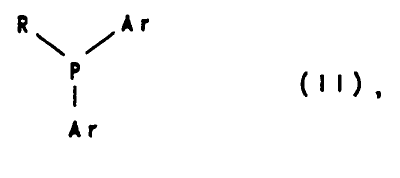 in which R is an alkyl radical having 1 to 6 carbon atoms, a cycloalkyl radical having 5 to 6 carbon atoms or Ar and each Ar has the abovementioned meaning.

2. The process as claimed in claim 1, characterized in that the substituted 1-halonaphthalene used is a 2-alkyl-1-halonaphthalene having 1 to 6 carbon atoms in the alkyl radical.

3. The process as claimed in claim 1 or 2, characterized in that the substituted 1-halonaphthalene used is a 2-methyl-1-halonaphthalene.

4. The process as claimed in one or more of claims 1 to 3, characterized in that the substituted 1-halonaphthalene used is a substituted 1-bromonaphthalene or 1-iodonaphthalene.

5. The process as claimed in one or more of claims 1 to 4, characterized in that the substituted 1-naphthylmagnesium halide used is a 2-alkyl-1-naphthylmagnesium halide having 1 to 6 carbon atoms in the alkyl radical.

6. The process as claimed in one or more of claims 1 to 5, characterized in that the substituted 1-naphthylmagnesium halide used is a 2-methyl-1-naphthalenemagnesium halide.

7. The process as claimed in one or more of claims 1 to 6, characterized in that the substituted 1-naphthylmagnesium halide used is a substituted 1-naphthylmagnesium bromide or 1-naphthylmagnesium iodide.

8. The process as claimed in one or more of claims 1 to 7, characterized in that the substituted 1-halonaphthalene and the substituted 1-naphthylmagnesium halide are reacted in a molar ratio of 1:(0.8 to 1.2), in particular 1:(0.9 to 1.1).

9. The process as claimed in one or more of claims 1 to 8, characterized in that the nonpolar solvent used is toluene, ethylbenzene, o-xylene, m-xylene, p-xylene, technical-grade mixtures of isomeric xylenes or mixtures thereof.

10. The process as claimed in one or more of claims 1 to 9, characterized in that the reaction is carried out at 50 to 120, in particular 70 to 100, °C.

11. The process as claimed in one or more of claims 1 to 10, characterized in that the palladium is used in accordance with a substituted 1-halonaphthalene/palladium molar ratio of (500 to 20):1, in particular (200 to 50):1.

12. The process as claimed in one or more of claims 1 to 11, characterized in that the palladium used is a Pd(II) compound.

13. The process as claimed in one or more of claims 1 to 12, characterized in that the Pd(II) compound used is Pd(II) acetate, Pd(II) sulfate, Pd(II) nitrate, Pd(II) chloride, Pd(II) acetylacetonate.

14. The process as claimed in one or more of claims 1 to 13, characterized in that the phosphine used is of the formula (I) in which m is an integer from 1 to 4, in particular 1 to 2, and n is an integer from 0 to 2.

15. The process as claimed in one or more of claims 1 to 14, characterized in that the phosphine used is of the formula (I) in which each Ar, independently of the others, is a phenyl, tolyl or fluorophenyl radical.

16. The process as claimed in one or more of claims 1 to 15, characterized in that the formula (I) phosphine used is 1,4-bis(diphenylphosphino)butane, 1,3-bis(diphenylphosphino)propane, 2,2'-bis(diphenylphosphinomethoxy)biphenyl or 2,2'-bis(diphenylphosphinomethoxy)-1,1'-binaphthyl, in particular 2,2'-bis(diphenylphosphinomethoxy)-1,1'-binaphthyl.

17. The process as claimed in one or more of claims 1 to 13, characterized in that the phosphine used is of the formula (II) in which R is Ar and Ar is a phenyl, tolyl or fluorophenyl radical.

18. The process as claimed in one or more of claims 1 to 13, characterized in that the formula (II) phosphine used is triphenylphosphine or tri-p-tolylphosphine.

19. The process as claimed in one or more of claims 1 to 18, characterized in that palladium and phosphine of the formula (I) are used in a molar ratio of 1:(0.8 to 2.0), in particular 1:(0.9 to 1.5), or palladium and phosphine of the formula (II) are used in a molar ratio of 1:(1.6 to 4.0), in particular 1:(1.8 to 3.0).

20. The process as claimed in one of claims 1 to 19, characterized in that palladium and phosphine of the formula (I) or (II) are used in the form of a palladium complex containing the phosphine of the formula (I) or (II).

21. The process as claimed in one or more of claims 1 to 20, characterized in that palladium and phosphine of the formula (I) or (II) are used in the form of bis[1,3 -bis(diphenylphosphino)propane]palladium, bis[2,2'-bis(diphenylphosphinomethoxy)-1,1'-binaphthyl]palladium or tetrakis(triphenylphosphine)palladium.

## Revendications

1. Procédé pour la préparation de 1,1'-binaphtylène, dans lequel on fait réagir un 1-halogénonaphtalène substitué en position 2 et éventuellement en une ou plusieurs des positions 3 à 7 par un radical alkyle, alcoxy ou dialkylamino avec 1 à 6 atomes de carbone dans le radical alkyle, et un halogénure de 1-naphtylmagnésium substitué en position 2 et éventuellement en une ou plusieurs des positions 3 à 7 par un radical alkyle, alcoxy ou dialkylamino avec 1 à 6 atomes de carbone dans le radical alkyle, dans un solvant non polaire, à 30 à 150°C, en présence de palladium et d'une phosphine de formule générale (I) : 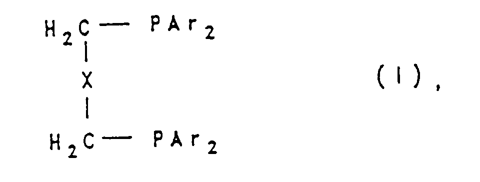 dans laquelle X représente (CH₂)ₘ et m est un nombre entier de 1 à 4 ou X représente un radical : 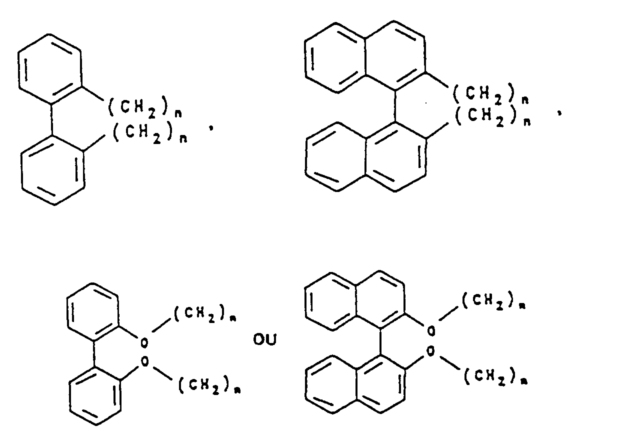 et n est un nombre entier de 0 à 2, les radicaux Ar, indépendamment l'un de l'autre, représentent chacun un radical phényle, tolyle, xylyle, fluorophényle ou trifluorométhylphényle, ou en présence de palladium et d'une phosphine de formule générale : 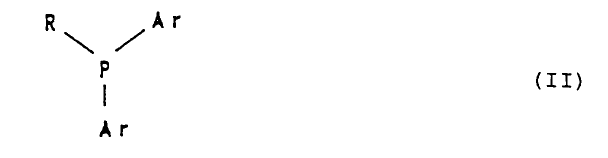 dans laquelle R représente un radical alkyle avec 1 à 6 atomes de carbone, un radical cycloalkyle avec 5 à 6 atomes de carbone ou Ar, et Ar a toujours la signification donnée précédemment.

2. Procédé selon la revendication 1, caractérisé en ce qu'on utilise comme 1-halogénonaphtalène substitué un 2-alkyl-1-halogénonaphtalène avec 1 à 6 atomes de carbone dans le radical alkyle.

3. Procédé selon la revendication 1 ou 2, caractérisé en ce qu'on utilise comme 1-halogénonaphtalène substitué un 2-méthyl-1-halogénonaphtalène

4. Procédé selon une ou plusieurs des revendications 1 à 3, caractérisé en ce qu'on utilise comme 1-halogénonaphtalène substitué un 1-bromonaphtalène ou un 1-iodonaphtalène substitué.

5. Procédé selon une ou plusieurs des revendications 1 à 4, caractérisé en ce qu'on utilise comme halogénure de 1-naphtylmagnésium substitué un halogénure de 2-alkyl-1-naphtylmagnésium avec 1 à 6 atomes de carbone dans le radical alkyle.

6. Procédé selon une ou plusieurs des revendications 1 à 5, caractérisé en ce qu'on utilise comme halogénure de 1-naphtylmagnésium substitué un halogénure de 2-méthyl-1-naphtalènemagnésium.

7. Procédé selon une ou plusieurs des revendications 1 à 6, caractérisé en ce qu'on utilise comme halogénure de 1-naphtylmagnésium substitué un bromure de 1-naphtylmagnésium ou un iodure de 1-naphtylmagnésium substitué.

8. Procédé selon une ou plusieurs des revendications 1 à 7, caractérisé en ce qu'on utilise le 1-halogénonaphtalène substitué et l'halogénure de 1-naphtylmagnésium substitué dans un rapport en moles de 1:(0,8 à 1,2), notamment de 1:(0,9 à 1,1).

9. Procédé selon une ou plusieurs des revendications 1 à 8, caractérisé en ce qu'on utilise comme solvant non polaire le toluène, l'éthylbenzène, l'o-xylène, le m-xylène, le p-xylène, des mélanges techniques d'isomères de xylène ou leurs mélanges.

10. Procédé selon une ou plusieurs des revendications 1 à 9, caractérisé en ce qu'on met en oeuvre la réaction à une température de 50 à 120°C, plus particulièrement de 70 à 100°C.

11. Procédé selon une ou plusieurs des revendications 1 à 10, caractérisé en ce qu'on utilise le palladium conformément à un rapport en moles du 1-halogénonaphtalène substitué au palladium de (500 à 20):1, notamment de (200 à 50):1.

12. Procédé selon une ou plusieurs des revendications 1 à 11, caractérisé en ce qu'on utilise comme palladium un composé de Pd(II).

13. Procédé selon une ou plusieurs des revendications 1 à 12, caractérisé en ce qu'on utilise comme composé de Pd(II) le Pd(II)-acétate, le Pd(II)-sulfate, le Pd(II)-nitrate, le Pd(II)-chlorure, le Pd(II)-acétylacétonate.

14. Procédé selon une ou plusieurs des revendications 1 à 13, caractérisé en ce qu'on utilise une phosphine de formule générale (I), dans laquelle m est un nombre entier de 1 à 4, plus particulièrement de 1 à 2, et n est un nombre entier de 0 à 2.

15. Procédé selon une ou plusieurs des revendications 1 à 14, caractérisé en ce qu'on utilise une phosphine de formule générale (I), dans laquelle les radicaux Ar indépendamment les uns des autres représentent chacun un radical phényle, tolyle ou fluorophényle.

16. Procédé selon une ou plusieurs des revendications 1 à 15, caractérisé en ce qu'on utilise comme phosphine de formule générale (I) le 1,4-bis(diphénylphosphino)butane, le 1,3-bis(diphénylphosphino)propane, le 2,2'-bis(diphénylphosphinométhoxy)biphényle ou le 2,2'-bis(diphénylphosphinométhoxy)-1,1'-binaphtyle, notamment le 2,2'-bis(diphénylphosphinométhoxy)-1,1'-binaphtyle.

17. Procédé selon une ou plusieurs des revendications 1 à 13, caractérisé en ce qu'on utilise une phosphine de formule générale (II) dans laquelle R représente Ar et Ar représente un radical phényle, tolyle ou fluorophényle.

18. Procédé selon une ou plusieurs des revendications 1 à 13, caractérisé en ce qu'on utilise en tant que phosphine de formule générale (II) la triphénylphosphine ou la tri-p-tolylphosphine.

19. Procédé selon une ou plusieurs des revendications 1 à 18, caractérisé en ce qu'on utilise le palladium et la phosphine de formule (I) dans un rapport en moles de 1:(0,8 à 2,0), notamment de 1:(0,9 à 1,5), ou le palladium et la phosphine de formule (II) dans un rapport en moles de 1:(1,6 à 4,0), notamment de 1:(1,8 à 3,0).

20. Procédé selon une ou plusieurs des revendications 1 à 19, caractérisé en ce qu'on utilise le palladium et la phosphine de formule (I) ou (II) sous forme d'un complexe de palladium contenant la phosphine de formule (I) ou (II).

21. Procédé selon une ou plusieurs des revendications 1 à 20, caractérisé en ce qu'on utilise le palladium et la phosphine de formule (I) ou (II) sous forme de palladium-bis-[1,3-bis(diphénylphosphino)propane], palladium-bis[2,2'-bis(diphénylphosphinométhoxy)-1,1'-binaphtyle] ou palladium-tétrakis-triphénylphosphine.
